(19) Europäisches Patentamt
European Patent Office
Office européen des brevets

(11) Numéro de publication : **0 097 096**
**B1**

# (12) FASCICULE DE BREVET EUROPÉEN

(45) Date de publication du fascicule du brevet : **28.08.85**

(51) Int. Cl.[4] : **C 07 C102/00**, C 07 C103/82// C07C117/00

(21) Numéro de dépôt : **83401166.0**

(22) Date de dépôt : **08.06.83**

## (54) Procédé de préparation de phénolamides.

(30) Priorité : **08.06.82 FR 8209932**

(43) Date de publication de la demande : **28.12.83 Bulletin 83/52**

(45) Mention de la délivrance du brevet : **28.08.85 Bulletin 85/35**

(84) Etats contractants désignés : **CH DE GB IT LI NL**

(56) Documents cités :
**DE-A- 2 331 262**
**DE-A- 2 429 779**

(73) Titulaire : **CENTRE NATIONAL DE LA RECHERCHE SCIENTIFIQUE (C.N.R.S.)**
**15, Quai Anatole France**
**F-75007 Paris (FR)**

**INSTITUT NATIONAL DE LA RECHERCHE AGRONOMIQUE (INRA)**
**149, rue de Grenelle**
**F-75341 Paris Cedex 07 (FR)**

(72) Inventeur : **Kunesch, Gerhard**
**7, allée clément Manet**
**F-91400 Orsay (FR)**
Inventeur : **Chuilon, née Monnier Sylvaine**
**14 Promenade Mona Lisa**
**F-78000 Versailles (FR)**
Inventeur : **Martin, Claude**
**Boîte 1, Cidex 49 bis, Savouges**
**F-21910 Saulon-la-Chapelle (FR)**
Inventeur : **Martin, née Tanguy Josette**
**Boîte 1, Cidex 49 bis, Savouges**
**F-21910 Saulon-la-Chapelle (FR)**

(74) Mandataire : **Corre, Jacques Denis Paul et al**
**Cabinet Regimbeau 26, Avenue Kléber**
**F-75116 Paris (FR)**

**EP 0 097 096 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne un procédé de préparation de phénolamides et les composés ainsi obtenus.

Les phénolamides préparés conformément au procédé de l'invention répondent à la formule

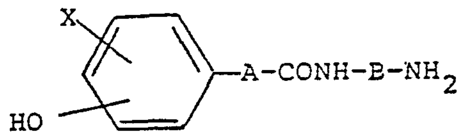

(I)

dans laquelle A et B sont des radicaux alcoylène ou alcénylène et X est un substituant éventuel, choisi parmi les halogènes et les radicaux —$NH_2$, —$R_1$ et —$OR_1$, $R_1$ étant l'hydrogène ou un groupe alcoyle inférieur.

Parmi les phénolamides préparés par le procédé de l'invention on peut citer plus particulièrement les composés de formule I ou A représente —CH=CH— et B—$(CH_2)_4$—.

Dans cette classe de composés, on peut citer encore plus particulièrement :
la férulylputrescine de formule

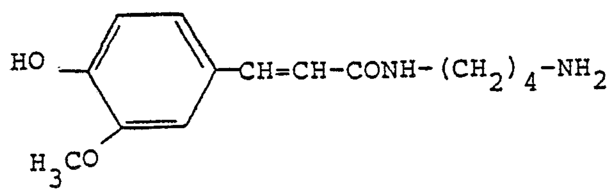

la p-coumarylputrescine de formule

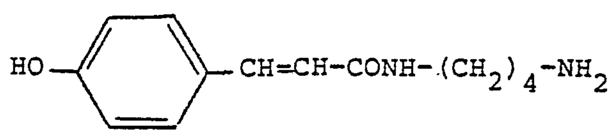

la caffeylputrescine de formule

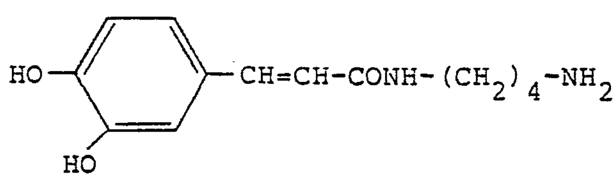

Ces composés ont été mis en évidence autour de la zone nécrotique d'hypersensibilité du *Nicotiana tabacum* variété Xanthi n.c au virus de la Mosaïque du Tabac, c'est-à-dire dans les cellules qui présentent un puissant pouvoir inhibiteur de multiplication virale.

Par la suite, il a été observé que divers phénolamides s'accumulaient dans les organes reproducteurs, ovaires et anthères.

La synthèse des phénolamides est très faible dans les plantes juvéniles et végétatives ; elle s'accélère brusquement peu de temps avant le passage à l'état reproducteur et prend une allure exponentielle lorsque le méristème passe de l'état végétatif à l'état reproducteur.

La relation qui existe entre la synthèse accélérée des phénolamides et le passage à l'état reproducteur a été confirmée de la façon suivante :

1) il est possible d'inhiber le passage à l'état reproducteur de nombreux végétaux : il suffit de les soumettre en permanence à une température élevée ; il n'y a jamais dans ce cas d'accélération exponentielle de la synthèse de phénolamides ;

2) certaines hybridations, ou certaines mutations, conduisent à l'obtention d'individus incapables de passer à l'état reproducteur ; il n'y a jamais non plus dans ce cas d'accélération de la synthèse de phénolamides ;

3) les anthères des plantes mâle-stériles (particulièrement recherchées en Amélioration des Plantes) sont dépourvues des phénolamides caractéristiques des anthères fertiles (Tabac, Féverole, Maïs, etc...).

En plus de leur activité antivirale, les phénolamides présentent des activités biologiques de type hormonal qui les apparentent à une nouvelle classe de facteurs de croissance.

C'est ainsi que l'on a pu démontrer que les phénolamides déclenchent dans certains cas un signal qui aboutit à une multiplication et une différenciation cellulaires.

Il en résulte que ces composés trouvent une application dans le domaine de la multiplication végétative *in vitro* et dans le domaine du génie génétique chez les végétaux supérieurs.

Devant les difficultés d'isolement des phénolamides naturels à partir de sources naturelles, il est apparu souhaitable, compte tenu des propriétés de cette classe de composés, de pouvoir les préparer en quantités suffisantes par des voies de synthèse chimique.

Les procédés classiques de formation d'amides conduisent toujours à l'obtention de mélange de diamides et de diamines contenant des quantités négligeables de monoamides, ce qui fait qu'ils ne peuvent être utilisés dans la pratique pour la synthèse des phénolamides précités.

Une publication récente décrit la synthèse de certains phénolamides par protection temporaire d'un des deux groupements $NH_2$ d'une diamine à l'aide de 18 — couronne — 6. Cette synthèse permet de préparer les composés considérés avec des rendements acceptables, mais une telle synthèse ne peut être utilisée en pratique compte tenu du prix élevé du réactif utilisé.

Le procédé qui fait l'objet de l'invention permet de préparer des phénolamides en quantités suffisantes et à des prix acceptables.

En outre il permet de récupérer directement le produit du milieu réactionnel, sans purification supplémentaire, ce qui constitue un avantage important du fait qu'il s'agit de produits particulièrement polaires.

Les composés répondant à la formule I ci-dessus sont préparés selon l'invention par les étapes consistant à :

a) faire réagir l'halogénure d'acide de formule

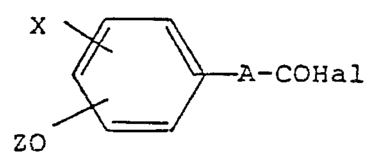

(II)

où A et X ont la signification indiquée ci-dessus, Hal représente un halogène et Z un groupe protecteur, avec un dérivé de formule

$$H_3N^+—B—Hal \qquad (III)$$

où B a la signification indiquée ci-dessus et Hal représente un halogène, en présence d'une amine tertiaire ;

b) faire réagir le composé de formule

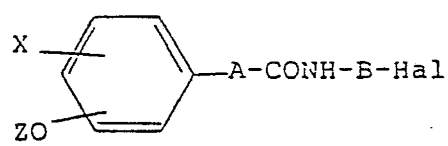

(IV)

ainsi obtenu où A, B, X, Z et Hal ont la signification indiquée ci-dessus, avec un azothydrate de formule

$$MeN_3 \qquad (V)$$

où Me représente un métal alcalin ou alcalino-terreux ;

c) faire réagir le composé de formule

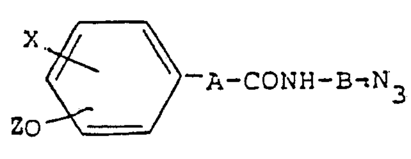

(VI)

ainsi obtenu où A, B, X et Z ont les significations indiquées ci-dessus, avec une base libérant le groupe protecteur Z ; et

d) soumettre le composé de formule

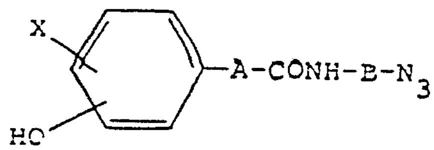

(VII)

ainsi obtenu, où A, B et X ont les significations données ci-dessus, à une hydrogénation catalytique.

L'halogénure d'acide de formule II sera avantageusement un chlorure d'acide obtenu, par exemple, par chloruration de l'acide correspondant avec le chlorure de thionyle. La protection de la fonction OH (ou des fonctions OH si X représente aussi un groupe OH) pourra être obtenue à l'aide de tout groupe protecteur Z approprié, par exemple par un groupe mésyle.

Dans ce cas on pourra effectuer la protection par réaction avec le chlorure de mésyle ($CH_3SO_2Cl$). Cette protection sera effectuée avantageusement avant la formation de l'halogénure d'acide.

Le dérivé $H_3N^+$—B—Hal de formule III peut être préparé par réaction du dérivé de formule

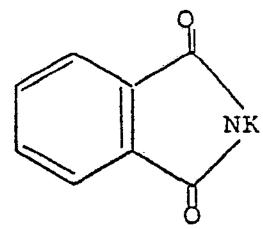

(VIII)

avec le dérivé de formule

Hal—B—Hal (IX)

où B et Hal ont la signification indiquée ci-dessus, pour donner le dérivé de formule

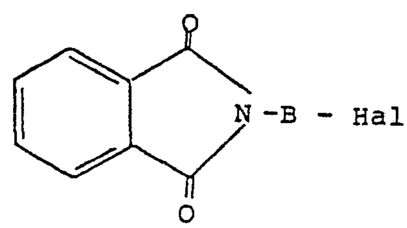

(X)

que l'on fait réagir avec l'acide de formule H—Hal.

Dans le schéma de réactions ci-dessus, l'halogène utilisé sera avantageusement le brome.

L'amine tertiaire utilisée dans l'étape (a) sera avantageusement constituée par la triéthylamine.

Le composé de formule V utilisée dans la réaction de l'étape (b) sera avantageusement constituée par l'azothydrate de sodium $NaN_3$.

Pour libérer le groupe protecteur Z, la base utilisée à l'étape (c) pourra être, par exemple, la potasse alcoolique.

L'hydrogénation de l'étape (d) est effectuée en présence d'un catalyseur approprié, par exemple du catalyseur de Lindlar (Pd/C).

D'autres caractéristiques et avantages de l'invention résulteront des exemples donnés uniquement à titre illustratif de la synthèse de phénolamides conformément au procédé de l'invention.

## Exemple 1

Préparation de la férulylputrescine

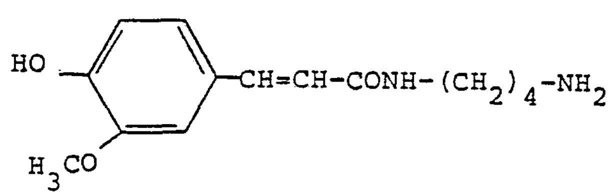

1.1 Préparation du chlorure de l'acide férulique mésylé

On prépare l'acide férulique mésylé conformément au schéma réactionnel suivant :

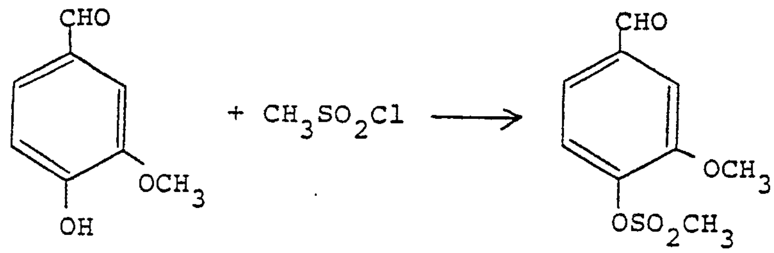

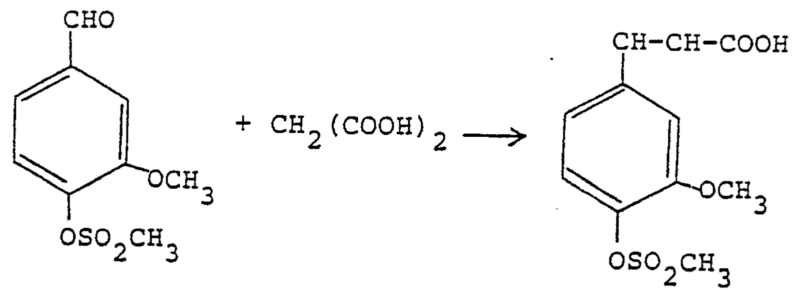

5,44 g (0,02 mole) de l'acide férulique mésylé ($C_{11}H_{12}O_6S$) ci-dessus sont placés dans un ballon de 250 ml surmonté d'un réfrigérant muni d'une garde remplie de gel de silice. On ajoute 30 ml de chlorure de thionyle fraîchement distillé et on chauffe à ébullition dans un bain d'huile maintenu à 100 °C pendant 2 h. On laisse refroidir à température ambiante et on évapore l'excès de réactif sous le vide de la trompe à eau. Le chlorure d'acide brut ainsi obtenu peut être utilisé sans autre purification. Le rendement est quantitatif.

1.2 Préparation du N-(Mésyl-4-féruloyl-)-1-amino-4-bromobutane.

On dissout le chlorure d'acide férulique mésylé dans 60 ml de chloroforme anhydre et on refroidit à 5 °C à l'aide d'un bain de glace. On y ajoute goutte à goutte 5 ml de triéthylamine par une ampoule à brome munie d'une garde, suivie d'une solution de 5,4 g de bromehydrate de 1-amino-4-bromobutane dans 40 ml de chloroforme anhydre pendant 10 minutes. On contrôle la fin de la réaction par chromatographie sur couche mince (solvant : acétate d'éthyle/chlorure de méthylène 50/50).

Le mélange réactionnel est transvasé dans une ampoule à décanter. La phase organique est lavée successivement à l'acide (HCl N), au bicarbonate de sodium saturé et enfin à l'eau distillée jusqu'à neutralité. On sèche sur sulfate de magnésium et on évapore sous le vide de la trompe à eau.

On obtient 8 g de produit brut suffisamment pur pour la réaction suivante.

Un échantillon a été purifié par chromatographie sur silicagel (acétate d'éthyle/chlorure de méthylène = 50/50).

$C_{15}H_{20}O_5N$ S Br ; F = 128-130 °C

Analyse

| | | | |
|---|---|---|---|
| Calculé : | C 44,23 | Trouvé : | 44,34 |
| | H 5,09 | | 4,96 |
| | N 3,58 | | 3,45 |

Spectre de masse : $M^+$ à m/e 405 + 407

pic de masse à m/e = 193

IR (KBr) : $\nu_{c=0}$ 1 660 $cm^{-1}$

RMN ($CDCl_3$, 60 MHz) : 3,90 ($-OCH_3$), 3,18 ($-SO_2CH_3$) ppm.

1.3 Préparation du N-(mésyl-4-férulyl)-1-amino-4-azidobutane.

11,06 g du bromure précédent sont chauffés à reflux dans 200 ml d'éthanol à 95 % dans un ballon de 500 ml surmonté d'un réfrigérant. (Température du bain d'huile : 100 °C). Après dissolution totale, on ajoute 3,64 g d'azothydrate de sodium dans une seule fois. On additionne ensuite de l'eau goutte à goutte jusqu'à dissolution totale de ce dernier (environ 2 ml). La transformation totale est vérifiée par CCM. Durée de la réaction : 2 h. Le mélange réactionnel est évaporé sous vide, repris dans le chlorure de méthylène et lavé à l'eau distillée deux fois (2 × 50 ml). Ensuite on sèche sur sulfate de magnésium et on évapore. (Rendement : 8,1 g = 73 %).

Le produit ainsi obtenu peut être utilisé sans purification.

Un échantillon a été purifié sur colonne de Silicagel pour les analyses.

$C_{15}H_{20}O_5N_4S$
Analyse

| | | | |
|---|---|---|---|
| Calculé : | C 48,9 | Trouvé : | 48,77 |
| | H 5,5 | | 5,64 |
| | N 15,2 | | 15,08 |

Spectre de masse : $M^+ = 368$,
pic de base à m/e = 192
IR (KBr) : $\nu N_3 = 2\,100\ cm^{-1}$
RMN ($CDCl_3$) = $\delta$ = 3,19 ($-SO_2CH_3$) 3,92 ($OCH_3$) ppm.

1.4 Préparation du N-Féruloyl-1-amino-4-azidobutane

1,6 g du produit mésylé précédent sont placés dans un ballon de 250 ml surmonté d'un réfrigérant. On purge l'atmosphère avec un courant d'argon et on maintient ensuite sous une légère pression d'argon. On ajoute à température ambiante 50 ml de potasse alcoolique 2N en une seule fois et on agite le mélange réactionnel à l'aide d'une agitation magnétique.

Après 15 h on évapore le solvant sous vide, on acidifie et on extrait au chlorure de méthylène. La phase organique est lavée à l'eau jusqu'à neutralité, puis séchée sur sulfate de magnésium. Après filtration et évaporation, on obtient 1,04 g d'une huile jaune pâle qui peut être purifiée par chromatographie sur colonne de gel de silicie (solvant chlorure de méthylène/acétate d'éthyle = 50/50). Rendement : 82 %.

$C_{14}H_{18}O_3N_4$
Analyse

| | | | |
|---|---|---|---|
| Calculé : | C 57,92 | Trouvé : | 57,65 |
| | H 6,25 | | 6,42 |
| | N 19,3 | | 19,05 |

IR ($CHCl_3$) : $\nu = 2\,100\ cm^{-1}$
RMN ($CDCl_3$) : $\delta$ ($OCH_3$) = 3,68/ppm.
Spectre de masse : $M^+$ à m/e = 290
pic de base à m/e = 177.

1.5 Préparation de la férulylputrescine.

2 g de N-Féruloyl-1-amino-4-azidobutane dissous dans 100 ml d'éthanol sont placés dans un appareil d'hydrogénation classique. On ajoute 100 mg de catalyseur de Lindlar (Pd/C). On purge l'appareil avec un courant d'hydrogène à deux reprises, puis on hydrogène en maintenant une agitation vigoureuse pendant 2 h. Après filtration du catalyseur, le solvant est évaporé sous vide. Le résidu cristallise lorsqu'on ajoute quelques gouttes d'alcool. Rendement : 1,78 g (97 %). Le produit peut être recristallisé d'un mélange éthanol/eau.

$C_{14}H_{20}O_3N_2$
Analyse

| | | | |
|---|---|---|---|
| Calculé : | C 63,61 | Trouvé : | 63,42 |
| | H 7,63 | | 7,70 |
| | N 10,6 | | 10,44 |

Spectre de masse : $M^+$ à m/e = 264
pic de base à m/e = 177
IR (KBr) : $\nu_{co} = 1\,640\ cm^{-1}$
RMN (DMSO-$d_6$) = $\delta$ ($OCH_3$) = 3,89 ppm.

Exemple 2

Préparation de la coumarylputrescine.

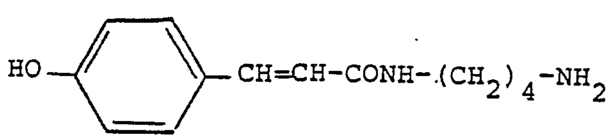

On prépare ce composé de façon analogue au composé de l'exemple I.

2.1 Préparation du chlorure de l'acide coumarique mésylé.

On prépare ce dérivé de façon analogue au chlorure de l'acide férulique mésylé (Exemple 1, paragraphe 1.1).

Le rendement est quantitatif.

2.2 Préparation du N-(Mésyl-4-coumaryl-)-1-amino-4-bromobutane.

$C_mH_{18}O_4S$ Br
Analyse

| | | | |
|---|---|---|---|
| Calculé : | C 44,68 | Trouvé : | 44,87 |
| | H 4,82 | | 4,90 |
| | N 3,72 | | 3,88 |

Spectre de masse $M^+$ à m/e = 375 et 377
pic de base à m/e = 225
IR (KBr) $\nu_{c=0}$ = 1 650 $cm^{-1}$
RMN ($CDCl_3$) = δ ($OSO_2CH_3$) 3,16 ppm
Rendement à partir de 4,8 g d'acide (0,02 mole)
6,9 g = 91 % brut

2.3 Préparation du N-(mésyl-4-coumaryl)-1-amino-4-azidobutane.

$C_{14}H_{18}O_4N_4S$
$M^+$ = 338
pic de base à m/e = 225
Analyse

| | | | |
|---|---|---|---|
| Calculé : | C 49,69 | Trouvé : | 49,80 |
| | H 5,36 | | 7,53 |
| | N 16,56 | | 16,40 |

IR (KBr) : $\nu_{N^3}$ = 2 100 $cm^{-1}$
$\nu_{co}$ = 1 650 $cm^{-1}$
RMN ($CDCl_3$) : δ ($OSO_2CH_3$m 3,15 ppm
Rendement : 82 % (6,9 g → 5,1 g)

2.4 Préparation du N-coumaryl-1-amino-4-azidobutane.

$C_{13}H_{16}O_2N_4$ (260)
Analyse

| | | | |
|---|---|---|---|
| Calculé : | C 59,98 | Trouvé : | 59,80 |
| | H 6,20 | | 6,18 |
| | N 21,53 | | 21,45 |

Spectre de masse : $M^+$ à m/e = 260
pic de base à m/e = 147
IR (KBr) $\nu_{N^3}$ = 2 100 $cm^{-1}$
$\nu_{co}$ = 1 660 $cm^{-1}$
RMN ($CDCl_3$)
Rendement : 64 % après chromatographie (5,1 → 2,51 g)

2.5 Préparation de la Coumarylputrescine :

$C_{13}H_{18}O_2N_2$ (260)
Spectre de masse : $M^+$ à m/e = 260
pic de base à m/e = 147
IR (KBr) = $\nu_{co}$ = 1 665 cm⁻1
Analyse

| | | | |
|---|---|---|---|
| Calculé : | C 66,64 | Trouvé : | 66,49 |
| | H 7,74 | | 7,79 |
| | N 11,96 | | 11,32 |

Rendement : 95 % (brut)

On peut, de façon correspondante aux exemples ci-dessus préparer la caffeylputrescine (en protégeant les deux fonctions OH) et d'autres composés de formule I.

Les phénolamides préparés par le procédé de l'invention ont des activités comparables à celles de la caféylputrescine et de la férulylputrescine en matière de multiplication virale et de différenciation cellulaire.

C'est ainsi que les phénolamides de l'invention présentent une activité virostatique en inhibant la multiplication des virus en cultures in vitro afin de régénérer des clones sains à partir d'individus entièrement contaminés.

Cette activité virostatique est applicable spécialement dans le domaine végétal, bien qu'elle puisse se manifester également à l'égard des virus animaux.

Les phénolamides de l'invention manifestent également une activité gaméticide dans le domaine végétal en inhibant le développement des gamettes mâles (grains de pollen). Cette activité gaméticide peut ainsi être mise à profit en production de semances pour la réalisation de $F_1$ (hybrides).

Les phénolamides de l'invention présentent en outre une activité herbicide en empêchant le développement normal des cellules végétales.

Il faut en outre signaler que les phénolamides de l'invention manifestent une activité « facteur de croissance ». Ils sont ainsi utilisables en culture in vitro pour l'obtention de nouvelles variétés de plantes cultivées et pour leur multiplication rapide in vitro (plantes horticoles, arbres fruitiers, essences forestières, etc.). Par ailleurs, ces phénolamides manifestent une activité régulatrice (excitatrice ou inhibitrice) de la division cellulaire. Ils trouvent donc une application antimitotique générale dans le domaine animal ou végétal, ce qui permet d'envisager une application dans la lutte contre le cancer.

Ces composés peuvent également avoir, dans les condititions naturelles, une action sur le développement des plantes cultivées ou non, en modifiant leur développement végétatif, leur fructification, leur tubérisation, leur entrée ou sortie de dormance.

Certains composés peuvent également contrôler les fermentations, la sporulation des champignons phytopathogènes, les cultures de bactéries, d'algues et de tous microorganismes à usage industriel.

## Revendications

1. Procédé de préparation de phénolamide de formule

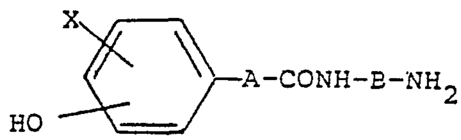

(I)

dans laquelle A et B sont des radicaux alcoylène ou alcénylène et X est un substituant éventuel choisi parmi les halogènes, les radicaux $—NH_2$, $—R_1$ et $—OR_1$, $R_1$ étant l'hydrogène ou un groupe alcoyle inférieur, caractérisé en ce qu'il comprend les étapes consistant à :

a) faire réagir l'halogénure d'acide de formule

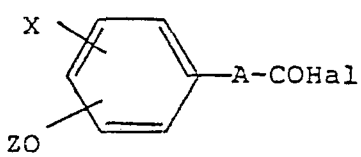

(II)

où A et X ont la signification indiquée ci-dessus, Hal représente un halogène et Z un groupe protecteur, avec un dérivé de formule

$$H_3N^+—B—Hal \qquad (III)$$

où B a la signification indiquée ci-dessus et Hal représente un halogène, en présence d'une amine tertiaire ;

b) faire réagir le composé de formule

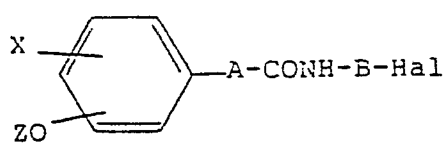

(IV)

ainsi obtenu où A, B, X, Z et Hal ont la signification indiquée ci-dessus, avec un azothydrate de formule

$$MeN_3 \qquad (V)$$

où Me représente un métal alcalin ou alcalinoterreux ;

c) faire réagir le composé de formule

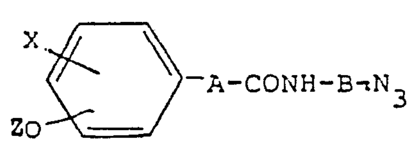

(VI)

ainsi obtenu où A, B, X et Z ont les significations indiquées ci-dessus, avec une base libérant le groupe protecteur Z ; et

d) soumettre le composé de formule

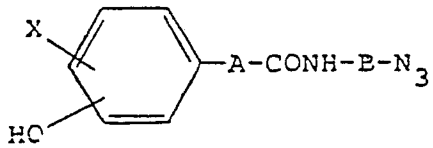

(VII)

ainsi obtenu, où A, B et X ont les significations données ci-dessus, à une hydrogénation catalytique.

2. Procédé selon la revendication 1, caractérisé en ce que le groupe protecteur Z est le groupe mésyle $—SO_2CH_3$.

3. Procédé selon la revendication 1, caractérisé en ce que le dérivé de formule III est préparé par réaction du dérivé de formule

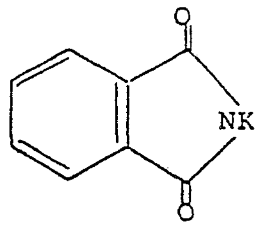

(VIII)

avec le dérivé de formule

Hal—B—Hal (IX)

où B et Hal ont la signification indiquée dans la revendication 1, pour donner le dérivé de formule

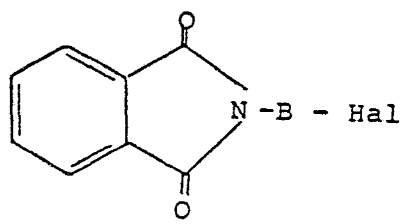

(X)

où B et Hal ont la signification indiquée ci-dessus, que l'on fait réagir avec l'acide de formule H-Hal.

4. Procédé selon la revendication 3, caractérisé en ce que Hal est Br.

5. Procédé selon la revendication 1, caractérisé en ce que l'amine tertiaire utilisée dans l'étage (a) est la triéthylamine.

6. Procédé selon la revendication 1, caractérisé en ce que le composé de formule V est l'azothydrate de sodium.

7. Procédé selon la revendication 1, caractérisé en ce que la base libérant le groupe protecteur Z, utilisée à l'étape (c), est la potasse alcoolique.

8. Procédé selon la revendication 1, caractérisé en ce que l'hydrogénation de l'étape (d) est effectuée en présence de catalyseur de Lindlar.

9. Procédé selon l'une des revendications 1 à 8, caractérisé en ce que l'on prépare le phénolamide de formule

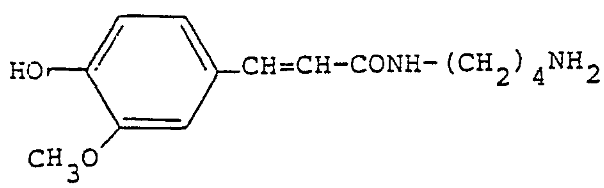

10. Procédé selon l'une des revendications 1 à 8, caractérisé en ce que l'on prépare le phénolamide de formule

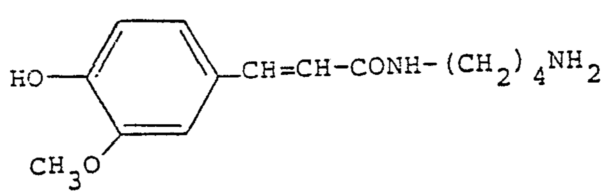

**Claims**

1. A process for the preparation of a phenolamide of the formula

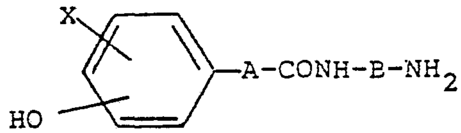 (I)

in which A and B are alkylene or alkenylene radicals and X is an optional substituent chosen from the halogens and the radicals $-NH_2$, $-R_1$ and $-OR_1$, $R_1$ being hydrogen or a lower alkyl group, which comprises the stages consisting in :

a) reacting the acid halide of the formula

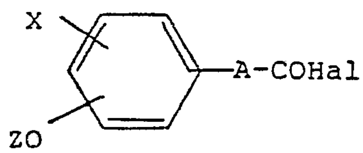 (II)

in which A and X have the meaning give above, Hal representents halogen and Z represents a protective group, with a derivative of the formula

$$H_3N^+-B-Hal \qquad (III)$$

in which B has the meaning given above and Hal represents halogen, in the presence of a tertiary amine ;

b) reacting the compound of the formula

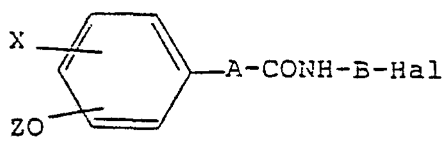 (IV)

thus obtained, where A, B, X, Z and Hal have the meaning given above, with an azide of the formula

$$MeN_3 \qquad (V)$$

in which Me represents an alkali metal or alkaline earth metal ;

c) reacting the compound of the formula

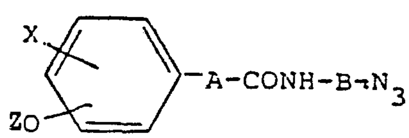 (VI)

thus obtained, in which A, B, X and Z have the meanings given above, with a base which liberates the protective group Z ; and

d) subjecting the compound of the formula

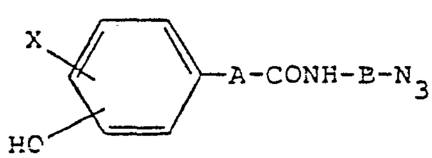 (VII)

thus obtained, in which A, B and X have the meanings given above, to catalytic hydrogenation.

2. The process as claimed in Claim 1, wherein the protective group Z is the mesyl group $-SO_2CH_3$.

3. The process as claimed in Claim 1, wherein the derivative of the formula III is prepared by reacting the derivative of the formula

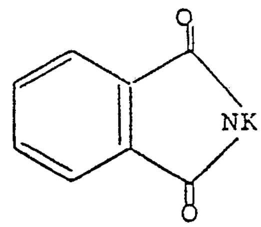

(VIII)

with the derivative of the formula

Hal—B—Hal (IX)

in which B and Hal have the meaning give the Claim 1, to give the derivative of the formula

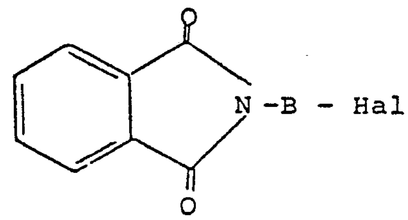

(X)

in which B and Hal have the meaning given above, which is reacted with the acid of the formula H-Hal.

4. The process as claimed in Claim 3, wherein Hal is Br.

5. The process as claimed in Claim 1, wherein the tertiary amine used in stage (a) is triethylamine.

6. The process as claimed in Claim 1, wherein the compound of the formula V is sodium azide.

7. The process as claimed in Claim 1, wherein the base which liberates the protective group Z and is used in stage (c) is alcoholic potassium hydroxide.

8. The process as claimed in Claim 1, wherein the hydrogenation in stage (d) is carried out in the presence of a Lindlar catalyst.

9. The process as claimed in any one of Claims 1 to 8, wherein the phenolamide of the formula

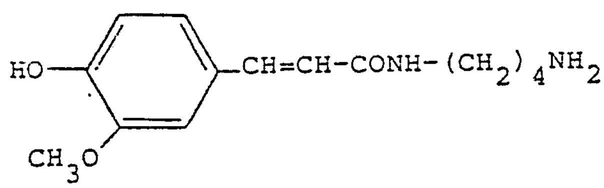

is prepared.

10. The process as claimed in any one of Claims 1 to 8, wherein the phenolamide of the formula

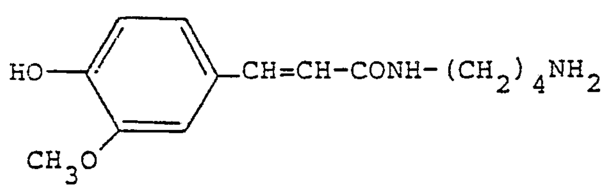

is prepared.

**Patentansprüche**

1. Verfahren zur Herstellung eines Phenolamids der Formel

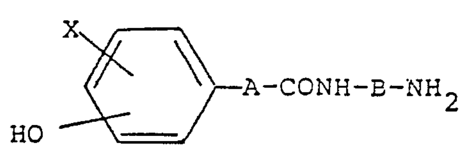

(I)

worin A und B Alkylen- oder Alkenylengruppen bedeuten und X gegebenenfalls ein Substituent ist, der aus Halogen, den Gruppen $—NH_2$, $—R_1$ und $—OR_1$ gewählt wird, wobei $R_1$ Wassestoff oder eine Niedrigalkylgruppe bedeutet, dadurch gekennzeichnet, daß es die folgenden Stufen umfaßt :

a) Umsetzen des Säurehalogenids der Formel

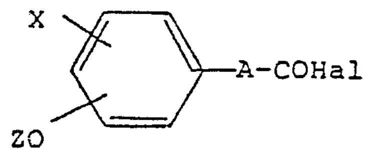

(II)

worin A und X die vorstehende Bedeutung besitzen, Hal ein Halogen und Z eine Schutzgruppe darstellen mit einem Derivat der Formel

$$H_3N^+—B—Hal \quad \text{(III)}$$

worin B die vorstehende Bedeutung besitzt und Hal ein Halogen darstellt, in Gegenwart eines tertiären Amins,

b) Umsetzen der so erhaltenen Verbindung der Formel

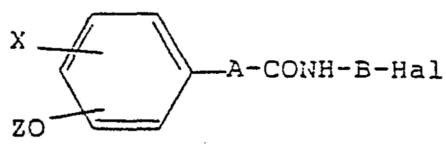

(IV)

worin A, B, X, Z und Ha1 die vorstehende Bedeutung besitzen, mit einem Azid der Formel

$$MeN_3 \quad \text{(V)}$$

worin M ein Alkali- oder Erdalkalimetall darstellt,

c) Umsetzen der so erhaltenen Verbindung der Formel

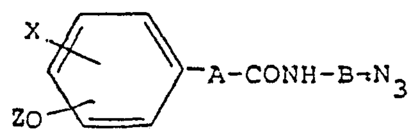

(VI)

worin A, B, X und Z die vorstehende Bedeutung besitzen, mit einer Base, die die Schutzgruppe Z freisetzt, und

d) Unterwerfen der so erhaltenen Verbindung der Formel

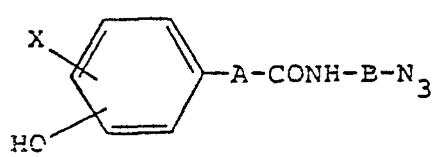

(VII)

worin A, B und X die vorstehende Bedeutung besitzen, einer katalytischen Hydrierung.

2. Verfahren nach Anspruch 1, dadurch gekennzeichnet, daß die Schutzgruppe Z die Mesylgruppe $—SO_2CH_3$ ist.

3. Verfahren nach Anspruch 1, dadurch gekennzeichnet, daß das Derivat der Formel III hergestellt wird durch Reaktion des Derivats der Formel

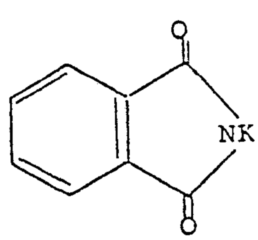

(VIII)

mit dem Derivat der Formel

Hal—B—HAl (IX)

worin B und Ha1 die in Anspruch 1 angegebene Bedeutung besitzen, um das Derivat der Formel

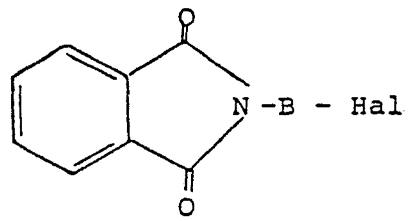

(X)

zu ergeben, worin B und Hal die vorstehende Bedeutung besitzen, welches mit der Säure der Formel H-Hal umgesetzt wird.

4. Verfahren nach Anspruch 3, dadurch gekennzeichnet, daß Hal Br ist.

5. Verfahren nach Anspruch 1, dadurch gekennzeichnet, daß das in Stufe (a) verwendete tertiäre Amin Triethylamin ist.

6. Verfahren nach Anspruch 1, dadurch gekennzeichnet, daß die Verbindung der Formel V Natriumazid ist.

7. Verfahren nach Anspruch 1, dadurch gekennzeichnet, daß die in Stufe (c) verwendete Base, die die Schutzgruppe Z freisetzt, alkoholisches Kaliumcarbonat ist.

8. Verfahren nach Anspruch 1, dadurch gekennzeichnet, daß die Hydrierung in Stufe (d) in Gegenwart eines Lindlar-Katalysators durchgeführt wird.

9. Verfahren nach einem der Ansprüche 1 bis 8, dadurch gekennzeichnet, daß das Phenolamid der Formel

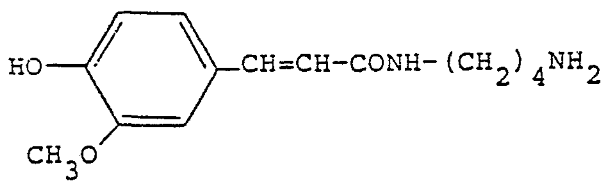

hergestellt wird.

10. Verfahren nach einem der Ansprüche 1 bis 8, dadurch gekennzeichnet, daß das Phenolamid der Formel

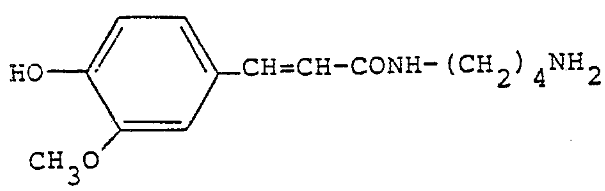

hergestellt wird.